(19) Europäisches Patentamt / European Patent Office / Office européen des brevets

(11) **EP 3 877 757 B1**

(12) **EUROPEAN PATENT SPECIFICATION**

(45) Date of publication and mention
of the grant of the patent:
**26.07.2023   Bulletin 2023/30**

(21) Application number: **19881863.5**

(22) Date of filing: **23.10.2019**

(51) International Patent Classification (IPC):
**G01N 27/02** *(2006.01)*    **D21D 1/30** *(2006.01)*
**G01N 33/34** *(2006.01)*    **H03H 7/01** *(2006.01)*

(52) Cooperative Patent Classification (CPC):
**G01N 27/028; D21D 1/30; G01N 33/343;**
G01N 27/026

(86) International application number:
**PCT/SE2019/051044**

(87) International publication number:
**WO 2020/096504 (14.05.2020 Gazette 2020/20)**

(54) **A FIBER CONCENTRATION PROFILE MEASURING APPARATUS**

VORRICHTUNG ZUR FASERKONZENTRATIONSPROFILMESSUNG

APPAREIL DE MESURE DE PROFIL DE CONCENTRATION DE FIBRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **05.11.2018   SE 1851372**

(43) Date of publication of application:
**15.09.2021   Bulletin 2021/37**

(73) Proprietor: **Daprox Ab**
**141 75 Kungens Kurva (SE)**

(72) Inventor: **ÅKERBLOM, Bengt**
**143 40 Vårby (SE)**

(74) Representative: **Zacco Sweden AB**
**P.O. Box 5581**
**Löjtnantsgatan 21**
**114 85 Stockholm (SE)**

(56) References cited:
EP-A2- 1 132 518          WO-A1-2005/083408
WO-A1-2015/065268       WO-A1-2015/065268
US-A- 3 104 373           US-A- 6 087 837
US-A1- 2004 214 549

## Description

### TECHNICAL FIELD

The present invention relates to a fiber concentration profile measuring apparatus according to the preamble of claim 1.

The present invention regards fiber concentration profile measuring apparatuses and pulp producing devices, such as a refiner. The present invention is mainly associated with the industry making paper, fiberboards etc. The present invention also relates to technical fields having an object to improve the accuracy of measurement of fiber concentration profiles in pulp producing devices and to improve energy efficiency in Thermo Mechanical Pulp TMP plants or similar by the provision of measurement of fiber concentration profiles being as correct and stable as possible during the production of pulp.

### BACKGROUND ART

Today different solutions exist to improve the measurement of fiber concentration profiles in so-called refiner zones of fiber pad distributions being processed between refiner discs of pulp producing devices or so-called refiners. New measurement techniques for measurement of fiber concentration profiles have been developed for promoting the ability to keep the quality of the pulp as consistent as possible by the development of fiber concentration profile measuring apparatuses that generate fiber concentration profiles being as correct and stable as possible. The fiber concentration profile measuring apparatus is configured to be mounted in the refiner discs of the pulp-producing device for measuring the fiber concentration profile in the fiber pad. The fiber concentration profile is preferably determined and measured in a direction from the centre of the refiner discs outwardly toward the perimeter of the refiner discs.

Paper making industry using refiners has interest in saving energy and there is a desire to balance energy consumption and fiber material refining process using such fiber concentration profile measuring apparatuses.

In research work, so-called sensor-rich systems are used for estimation of fiber consistency.

However, such systems are bulky and require complex software applications executing data from temperature sensors, motor loads, dilution water feeding rate, inlet temperature and pressure, material properties etc. The use of temperature data is hazardous since the temperature per se does not indicate the refiner pad consistency and fiber concentration.

### SUMMARY OF THE INVENTION

The inventor of the present invention disclosed herein has presented one way to overcome the above-mentioned problems according to an alternative solution shown in the document WO 2015/065268 A1. The document WO 2015/065268 A1 reveals a fiber concentration profile measuring apparatus comprising conductor bodies configured for detecting the degree of fiber concentration of the fiber pad material being pulped. Each conductor body of a stationary refiner disc exhibits an electric contact surface arranged opposite the rotating refiner disc. A distance between the refiner discs forms a grinding gap. The grinding gap embodies the fiber pad material. The actual electrical resistivity and/or electrical conductance of the fiber pad material will determine the electrical resistance, which being measured by each conductor body one at a time. The actual fiber concentration at each position is thus derived from the electrical resistivity and/or electrical conductance of the current fed through each individual conductor body. Software is used by a control unit for determination of the fiber concentration profile. The conductor bodies are connected in series with a respective series resonance circuit being individually adapted to give free passage for the electric current to a certain frequency. It has been shown that the fiber concentration profile measuring apparatus disclosed in WO 2015/065268 A1 facilitates the handling of the fiber concentration profile measuring apparatus during service and maintenance and during mounting of it to the refiner disc in an effective manner. Cost-effective handling of the apparatus is thus achieved by that the number of lead wires coming from the fiber concentration profile measuring apparatus is reduced simply by earmarking each individual conductor body by arranging each one of them in series with a specific series resonance circuit.

The fiber pad material thus may serve as a conductor by itself and exhibits a certain conductivity/electrical resistivity depending upon the actual fiber concentration and/or the actual moisture content and from what the actual gaseous or liquid state of the water content or other gaseous phase of the fiber pad material.

The determination of the fiber concentration of the fiber pad material may be performed over a segment of the refiner discs seen in a direction from the centre of the refiner discs towards the perimeter of the refiner discs.

The rate of changing the direction of the AC may define the frequency, which is generated by a signal generator associated with the power source.

An individual signature of each conductor body is made by coupling an individual conductor body to the specific series resonance circuit, which exhibits a different resonance frequency that the others due to e.g. its resistance, inductance, and/or capacitance. The signal generator alternatively generates electrical output waveforms over a pre-determined range of frequencies and the power source is controlled by a frequency sweep controller as to execute the frequency sweep. The fiber concentration profile measuring apparatus disclosed in WO 2015/065268 A1 is of high technical value to the technical field.

US6087837 relates to systems for controlling continuous sheetmaking systems and, more specifically, to impedance-type sensors for measuring the fiber weight of wetstock in a papermaking machine. If EP1132518 shows a rotary disk refiner for refining fibrous pulp in a liquid stock using a plurality of resistive-type sensors.

However, there is a desire to further develop and simplify known fiber concentration profile measuring apparatuses for determination of the fiber concentration profile of the fiber pad material being pulped.

It is desirable save further energy in the paper making industry by providing even more exact fiber concentration profile measurement.

It is desirable to produce a high quality pulp by providing even more exact fiber concentration profile measurement.

This has been achieved by the fiber concentration profile measuring apparatus being defined in the introduction and being characterized by the features of the characterizing part of claim 1. By sweeping the frequency (e.g. 150-350 kHz) of the secondary electric current, the specific electrical resistivity and/or conductivity and/or impedance of the primary electric current fed through the fiber pad material in different positions along the radius of a refiner disc can be measured by the impedance measuring unit providing impedance values to a control unit of the fiber concentration profile measuring apparatus for converting the impedance values into actual fiber concentration profile values.

Alternatively, the secondary power source is configured to generate a secondary electric current injected with a range of frequencies, wherein a first series resonance circuit is configured at a first frequency to give free passage for the secondary electric current to a first electronic switching device and a second series resonance circuit is configured at a second frequency to give free passage for the secondary current to a second electronic switching device.

Alternatively, each specific electronic switching device is coupled in series with a specific individual series resonance circuit, for switching each electronic switching device to on-state independently of each other, i.e. each individual electronic switching device will switch to on-state depending on which frequency the specific individual series resonance circuit being adapted to give free passage for the secondary current.

Alternatively, the electronic switching device comprises a semiconductor relay or solid-state relay.

Alternatively, the second electric contact surface is formed by a rotor refiner disc.

Alternatively, the fiber concentration profile measuring apparatus is mounted in a pulp producing device or refiner apparatus configured to pulp fiber pad material.

Alternatively, the primary AC power source may comprise the secondary power source.

Alternatively, the fiber concentration profile measuring apparatus comprises; a first conductor body coupled to the primary AC power source via a first electronic switching device; a second conductor body coupled to the primary AC power source via a second electronic switching device; a third conductor body coupled to the primary AC power source via a third electronic switching device; the first conductor body exhibits a first electric contact surface adapted to transfer the primary electric current from the primary AC power source to the second electric contact surface via the fiber pad material being pulped; the second conductor body exhibits a second electric contact surface adapted to transfer the primary electric current from the primary AC power source to the second electric contact surface via the fiber pad material being pulped; the third conductor body exhibits a third electric contact surface adapted to transfer the primary electric current from the primary AC power source to the second electric contact surface via the fiber pad material being pulped; the first conductor body is coupled in series with the first electronic switching device; the second conductor body is coupled in series with the second electronic switching device; the third conductor body is coupled in series with the first third electronic switching device; the first electronic switching device is configured to switch between first on-state and first off-state so that the primary AC power source can feed or not feed the primary electric current to the first conductor body; the second electronic switching device is configured to switch between second on-state and second off-state so that the primary AC power source can feed or not feed the primary electric current to the second conductor body; the third electronic switching device is configured to switch between third on-state and third off-state so that the primary AC power source can feed or not feed the primary electric current to the third conductor body.

The secondary power source may be configured to generate a secondary electric current injected with a range of frequencies.

The secondary power source may be coupled to the first electronic switching device via a first series resonance circuit being individually adapted to give free passage for the secondary electric current at a first frequency, whereby the secondary electric current can affect the first electronic switching device to switch to said first on-state so that the primary electric current is fed from the primary AC power source to the first conductor body implying that the primary electric current is fed from the first electric contact surface to the second electric contact surface via the fiber pad material.

Alternatively, the impedance-measuring unit may determine a first impedance value and a control unit of the fiber concentration profile measuring apparatus derives the fiber concentration from the first impedance value of the primary electric current fed through the first conductor body.

Alternatively, the first impedance value represents the actual property of the fiber pad material between the refiner discs at a first position of the first conductor body.

The secondary power source is coupled to the second electronic switching device via a second series resonance circuit being individually adapted to give free passage for the secondary electric current at a second frequency, whereby the secondary electric current can affect the second electronic switching device to switch to said second on-state so that the primary electric current is fed from the primary AC power source to the second conductor body implying that the primary electric current is fed from the second electric contact surface to the second electric contact surface via the fiber pad material.

Alternatively, a second impedance value is determined by the impedance-measuring unit and the control unit derives the fiber concentration from the second impedance value of the primary electric current.

Alternatively, the second impedance value represents the actual property of the fiber pad material between the refiner discs at a second position of the second conductor body.

The secondary power source is coupled to the third electronic switching device via a third series resonance circuit being individually adapted to give free passage for the secondary electric current at a third frequency, whereby the secondary electric current can affect the third electronic switching device to switch to said third on-state so that the primary electric current is fed from the primary AC power source to the third conductor body implying that the primary electric current is fed from the third electric contact surface to the second electric contact surface via the fiber pad material.

Alternatively, a third impedance value is determined by the impedance measuring unit and the control unit derives the fiber concentration from the third impedance value.

Alternatively, the third impedance value represents the actual property of the fiber pad material between the refiner discs at a third position of the third conductor body.

For each conductor body and for each position to be measured regarding the fiber pad material property (e.g. by measuring the impedance of the primary electric current fed through the fiber pad material at the specific position), there is thus provided a primary electric circuit.

Alternatively, a primary electric circuit comprises the first conductor body coupled to the primary AC power source via the first electronic switching device, which in turn is coupled to the secondary power source via the first series resonance circuit (adapted to give free passage for the secondary current at a first frequency to the first electronic switching device for making the first on-state) coupled in series between the secondary power source and the first electronic switching device and thus controlling the first electronic switching device to switch to the first on-state at the first frequency for permitting the primary electric current to be fed from the primary AC power source to the first conductor body.

Alternatively, the primary electric circuit comprises the second conductor body coupled to the primary AC power source via the second electronic switching device, which in turn is coupled to the secondary power source via the second series resonance circuit (adapted to give free passage for the secondary current at a second frequency to the second electronic switching device for making the second on-state) coupled in series between the secondary power source and the second electronic switching device and thus controlling the second electronic switching device to switch to the second on-state at the second frequency for permitting the primary electric current to be fed from the primary AC power source to the second conductor body.

Alternatively, the primary electric circuit comprises the third conductor body coupled to the primary AC power source via the third electronic switching device, which in turn is coupled to the secondary power source via the third series resonance circuit (adapted to give free passage for the secondary current at a third frequency to the third electronic switching device for making the third on-state) coupled in series between the secondary power source and the third electronic switching device and thus controlling the third electronic switching device to switch to the third on-state at the third frequency for permitting the primary electric current to be fed from the primary AC power source to the third conductor body.

Alternatively, the above-mentioned ordinal number "e.g. second, third etc." can also be "fourth", "fifth", "sixth", "seventh", "eight", "ninth" associated with each conductor body and/or each electric measuring circuit, depending upon how many conductor bodies being used in the primary electric circuit of the fiber concentration profile measuring apparatus for proving said fiber concentration profile.

Alternatively, the number of contact bodies is any of 3 to 100.

Alternatively, the primary AC power source of the fiber concentration profile measuring apparatus is provided with a primary frequency generator configured to generate a suitable frequency, e. g 50 kHz, of the primary electric current.

Alternatively, the primary frequency generator is configured to generate a frequency of 50 Hz or any frequency that generates a stable current at narrow bandwidth.

In such way is achieved that the primary electric current passing through the conductor bodies is stable at one specific frequency, which also eliminates the need of running the fiber concentration profile measuring apparatus on high or low bandwidth

In such way is eliminated the use of an AC power source not feeding current linearly over the range of high and low bandwidth - i.e. from high to low frequency - for determining the impedance/resistance of the fiber pad material at the position of each conductor body.

The primary AC power source may be configured to generate one fixed frequency, which will produce a stable primary electric current, which in turn will produce reliable impedance values of the conductor bodies, and reliable fiber concentration profile values converted by the control unit.

By using a primary AC power source providing a primary electric current comprising a fixed frequency (e.g. 50 kHz), there will be a stable current to be measured in regard to the resistance or impedance.

Alternatively, the primary AC power source is configured to generate an AC primary electric current over a transformer comprising a first winding and a secondary winding arranged in the fiber concentration profile measuring apparatus.

The conductor bodies may be connected in series with the respective electronic switching device, each of which is configured to close or open dependent on the control signal from the respective SRC.

The fiber concentration profile measuring apparatus may comprise a control unit.

It will thus be possible to control the performance of the pulp-producing device from actual fiber concentration profile measurement.

In such a way, the number of lead wires coming from the fiber concentration profile measuring apparatus is reduced.

In such way there is achieved a way to eliminate the dependence of using a common AC power source and signal generator for feeding the current to the respective conductor body. The use of a common AC power and signal generator feeding a current to the respective conductor body may generate disturbed current in a non-linear manner over the used frequency range. It has been shown that it is difficult to generate the current accurately at high and low bandwidth. Such current may otherwise affect the performance of the fiber concentration profile measuring apparatus.

Thereby is achieved that specific values of the measured conductivity and/or electrical resistivity over the radial direction of the refiner discs, partly or entirely, may be calculated by a control unit for executing conversion of the impedance/resistance into actual fiber concentration and/or temperature.

The control unit may display a curve profile indicating the actual changes of fiber concentration and/or temperature from the electrical material property of the fiber pad during production.

The varying of electrical material properties of the fiber pad are indicated by each earmarked conductor body.

The actual value of conductivity and/or electrical resistivity may be measured in each position of each conductor body for providing a fiber concentration and/or temperature curve profile and/or registration of impedance.

The temperature may be derived by the control unit from signals fed from a resistance temperature detector (e.g. a PT100 element) or resistance temperature detectors arranged to the fiber concentration profile measuring apparatus.

The actual values sensed and calculated by the control unit may be compared with set point fiber concentration values by means the control unit.

The control unit may be coupled to the fiber concentration profile measuring apparatus and configured to detect alteration of the electrical resistivity and/or conductivity and/or impedance of the fiber pad material.

A primary circuit may comprise a primary signal generator of the primary AC power source, the impedance-measuring unit, the conductor bodies and conductor wires.

A secondary circuit may comprise a secondary signal generator of a secondary power source, RCEs, and conductor wires.

The respective electronic switching device may serve as a component common for both the primary circuit and secondary circuit.

By sweeping the frequency of the secondary electric current in the secondary circuit, the specific electrical resistivity and/or conductivity of the fiber pad material in different positions along the radius of the refiner disc can be measured by the primary circuit.

Preferably, the second electric contact surface is formed by a rotor refiner disc.

In such way is achieved that a first grinding surface of the rotor refiner disc may be used as a second electric contact surface of the fiber concentration profile measuring apparatus.

The electrical coupling for the primary electrical current may led from the second electric contact surface via the axis of the rotor refiner disc to earth.

A slip contact may be mounted between the rotary refiner disc axis and an earth connected refiner disc fundament used for contacting the rotary refiner disc to earth.

The primary electrical current may be fed between the respective first electric contact surface of each conductor body and the rotor refiner disc.

The respective conductor body may be positioned in the stator refiner disc in such way that the respective first electrical contact surface is flush or at least adjacent with a second grinding surface of the stator refiner disc.

Alternatively, the conductor bodies are connected in series with a respective electronic switching device, each of which is configured to close or open dependent on a control signal from a respective series resonance circuit.

Alternatively, the respective series resonance circuit being individually adapted to give free passage for the secondary electric current to a certain frequency.

This is made by earmarking each individual conductor body by arranging each conductor body in series with a respective electronic switching device.

Alternatively, each electronic switching device is in turn coupled to a respective series resonance circuit having a certain frequency at which the secondary electric current is given passage for closing the associated electronic switching device (the secondary electric current controls the electronic switching device to close).

The respective electronic switching device thus being controlled to close for permitting the primary electric current to pass to the associated conductor body.

The respective conductor body may be connected in series with the respective electronic switching device, each of which is configured to close or open (on-state or off-state) in dependency on the control signal (to give free passage of current or hinder passage of current) from the respective series resonance circuits.

By closing the respective electronic switching device independently of each other, the respective "earmarked" conductor body will be put into use for feeding the primary electric current from the primary AC power source through the fiber pad at a specific position of the refiner discs processing the fiber pad (the "earmarked" conductor bodies may be positioned along an imaginary line drawn in a direction from the centre of the refiner discs towards the perimeter of the refiner discs).

Alternatively, when a specific electronic switching device is closed for permitting the primary electric current to pass, the other electronic switching devices are open for disconnecting the other conductor bodies from the primary AC power source.

Alternatively, the other electronic switching devices (not yet closed by the secondary electric current) are open, as they are not affected by any secondary electric current.

The other series resonance circuits, each of which having a specific frequency at which the secondary electric current is given passage.

By using a primary AC power source providing a primary electric current comprising a fixed frequency (e.g. 50 kHz), there will be a stable current to be measured in regard to the resistance or impedance.

The primary AC power source may be coupled to an impedance measuring unit or comprising an impedance measuring unit configured to measure variations of the impedance of the primary electric current.

By using a secondary power source providing varying frequencies (e.g. 150-500 kHz) there has been achieved a way to separately fed a secondary electric current from the primary AC power source to each "earmarked" conductor body one by one.

By sweeping the frequency of the secondary electric current from the secondary power source, the specific electrical resistivity and/or conductivity of the fiber pad in different positions along the radius of the refiner disc can be measured.

By injecting various frequencies of the secondary electric current and controlling the respective electronic switching device be means of the respective specific series resonance circuit for feeding the stable primary electric current to each "earmarked" conductor body coupled to the respective electronic switching device.

There is thus possible to achieve individual signatures of each conductor body.

The rate of changing the direction of the alternating secondary electric current defines a specific frequency of the frequencies of the secondary electric current.

The frequency may be provided by a signal generator associated with an AC secondary power source (e. g. a secondary transformer driver).

An individual signature may be made by "earmarking" each conductor body by coupling the respective conductor body to the respective electronic switching device, each of which being coupled to the respective specific series resonance circuit each configured to give free passage for the secondary electric current at a certain frequency providing the on-state (closed electronic switching device).

The respective specific series resonance circuit may be configured to a different resonance frequency than the others due to certain RLC (resistance of e.g. lead wire, inductance, and capacitance) of each specific series resonance circuit.

The respective specific series resonance circuit (each being configured to give free passage of the secondary electric current at a specific frequency) may respond selectively to signals of the specific frequency.

The respective specific series resonance circuit may comprise a lead wire, inductor, capacitor connected in series.

Thus, by injecting various frequencies to the secondary electric current is it possible to achieve individual signatures of each conductor body position, since each specific series resonance circuit may close the respective electronic switching device for feeding the primary electric current to each "earmarked" conductor body one by one.

The plurality of conductor bodies, the respective series resonance circuits and the electronic switching devices, may be encompassed in an elongated casing or housing of the fiber concentration profile measuring apparatus.

In such way is achieved a stable primary electric current (from the primary transformer driver) which is fed through the fiber pad at different positions, at the same time as the number of electronic components are reduced and the number of electrical wires or conductors coupled to the elongated casing or housing.

Alternatively, the secondary signal generator of the secondary power source is adapted to generate a frequency sweep from e. g. 150 kHz - 500 kHz to the secondary circuit.

The secondary signal generator alternatively generates electrical output waveforms over a preferable range of frequencies (e.g. 150-500 kHz).

The secondary power source may be controlled by a frequency sweep controller as to execute the frequency sweep.

The frequency of the driving voltage of the secondary electric circuit may be swept from a predetermined upper frequency 500 kHz to a predetermined lower frequency 150 kHz by means of the secondary signal generator.

The secondary signal generator may include a function of automatically and repetitively sweeping the frequency of output waveforms by means of a voltage controlled oscillator between two defined limits.

Thus, by sweeping frequency of the secondary electric current over a specific frequency range (for example 150-500 kHz) it is possible to cover the different individually determined and adapted (set) resonance frequencies of the various SRCs (for example; a first SRC is set to 150 kHz, a second SRC is set to 200 kHz, a third SRC is set to 250 kHz, a fourth SRC is set to 300 kHz, a fifth SRC is set to 350 kHz, a sixth SRC is set to 400 kHz, a seventh SRC is set to 450 kHz and an eight SRC is set to 500 kHz.

The conductor bodies may be positioned along a straight line in an elongated housing, wherein the first electric contact surfaces are coplanar with the grinding surface of the refiner disc (stator disc) in which the elongated housing is mounted.

In such way is achieved that simplified handling for service personnel is provided.

The area of the first electric contact surface, the width of the grinding gap (the distance between the refiner discs), and the actual electrical resistivity $\rho$ of the fiber pad material determine the electrical resistance R, which being measured continuously for detecting the alteration of the electrical impedance of the fiber pad.

The control unit may be configured to convert the electrical impedance of the primary electric current into actual fiber concentration, e.g. by empirically implementation, of each position and thus providing the fiber concentration profile.

The fiber pad material serves as a conductor by itself and exhibits a certain conductivity/electrical resistivity/electrical impedance depending upon the actual fiber concentration and/or the actual moisture content and from what the actual gaseous or liquid state of the water content or other gaseous phase of the fiber pad material.

In such way is achieved a fiber concentration profile measuring apparatus that uses just a few number of cables drawn from the elongated body or the elongated casing/housing.

By measuring the conductivity (or resistance or resistivity or impedance) there is provided a way to determine whether the fiber pad material is in liquid state or in a gaseous state as the gaseous state has a high resistivity relative the liquid state.

The control unit may calculate the fiber concentration of the fiber pad material for each position of the respective conductor body from detection of the actual electrical resistivity of the fiber pad material in each position of the respective conductor body.

The control unit may be configured to calculate the fiber concentration profile of the fiber pad material in the grinding gap from the fiber concentration of the fiber pad material for each position of the respective conductor body.

The control unit may be configured to detect alteration of the electrical resistivity and/or conductivity and/or impedance of the fiber pad material by measuring the primary electric current.

In such way is achieved that the control unit being able to calculate and provide a fiber concentration profile illustrated radially over the refiner disc on a display or other user interface.

The electrical resistivity may be called electrical impedance.

The resistance may also be called electrical resistance and associated with electrical impedance, being electrical resistance for an alternating current and measured in ohm.

The primary AC power source may be configured to generate one single fixed frequency, which will produce a stable primary electric current, which in turn will produce reliable impedance values of the primary electric current passing the conductor bodies one by one.

BRIEF DESCRIPTION OF THE DRAWINGS

The present invention will now be described by way of examples with references to the accompanying schematic drawings, of which:

FIG. 1 illustrates a fiber concentration profile measuring sensor according to a first example of the invention;

FIG. 2 illustrates a stator disc comprising a fiber concentration profile measuring sensor according to a second example of the invention;

FIG. 3 illustrates a fiber concentration profile measuring sensor according to a third example of the invention;

FIG. 4 illustrates a fiber concentration profile measuring sensor according to a fourth example of the invention; and

FIG. 5 illustrates a fiber concentration profile measuring sensor according to a fifth example of the invention.

DETAILED DESCRIPTION

Hereinafter, embodiments of the present invention will be described in detail with reference to the accompanying drawings, wherein for the sake of clarity and understanding of the invention some details of no importance are deleted from the drawings.

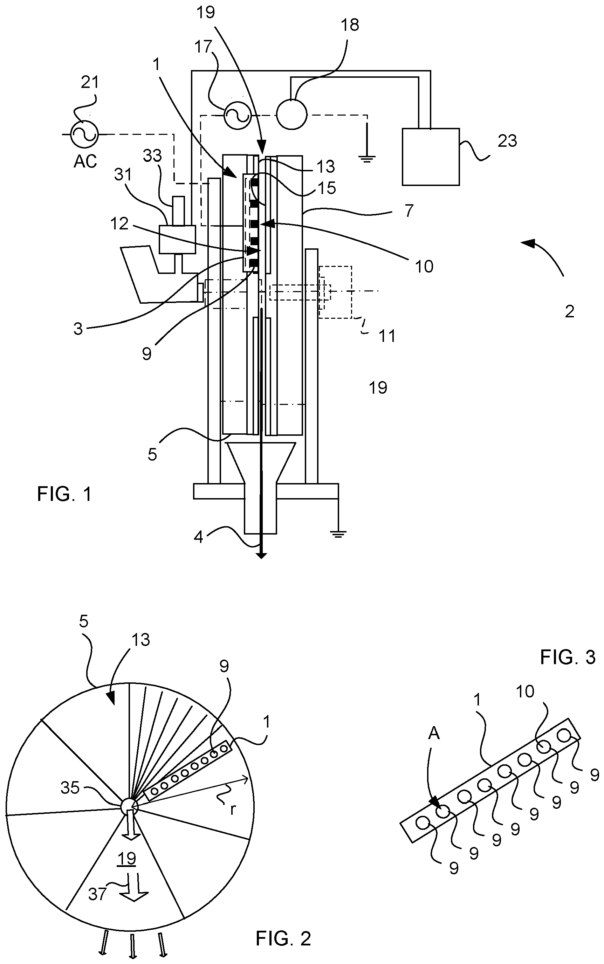

FIG. 1 illustrates a refiner 2 comprising a fiber concentration profile measuring sensor 1 according to a first example. The refiner 2 is configured for grinding a fiber pad material (not shown) into a pulp 4. The fiber pad material is grinded between a stator disc 5 and a rotor disc 7. The fiber concentration profile measuring sensor 1 is mounted in the stator disc 5. A motor 11 drives the rotor disc 7.

An elongated casing 3 of the fiber concentration profile measuring sensor 1 comprises five conductor bodies 9 each exhibiting a first electric contact surface 10 being configured to feed a primary electric current through the fiber pad material to a second electric contact surface 12 formed by the rotor disc 7. The conductor bodies 9 are coupled to a primary AC power source 17. The respective first electric contact surface 10 is configured to transfer a primary electric current from the primary AC power source 17 to the second electric contact surface 12 via the fiber pad material being pulped in a grinding gap 19 formed between a stationary grinding surface 13 and a rotary grinding surface 15.

The primary AC power source 17 is coupled to an impedance measuring instrument 18 configured to measure variations of the impedance of the primary electric current.

The impedance-measuring instrument 18 may be replaced by any type of impedance measuring instrument or other suitable instrument, e.g. an ampere meter, for registration of the variations of the resistance of the primary electric current.

Each conductor body 9 is coupled in series with a respective electronic switching device (not shown), each of which is configured to switch between on- and off-state for feeding or not feeding the primary electric current to the respective conductor body 9.

A secondary power source 21 is configured to generate a secondary electric current injected with a range of frequencies.

The secondary power source 21 is coupled to each electronic switching device (not shown) via a respective series resonance circuit (not shown) being individually adapted to give free passage for the secondary electric current at a certain frequency providing said on-state of the respective electronic switching device one by one.

The fiber concentration profile measuring sensor 1 may comprise a processor 23 configured to detect alteration of the electrical resistivity and/or conductivity of the fiber pad material by measuring the impedance of the primary electric current fed through the fiber pad material at the position of each conductor body 9.

The voltage of the primary electric current fed by the primary AC power source 17 may be constant.

The fiber concentration profile measuring sensor 1 may be electrically insulated arranged at the stator disc 5 preventing any current overflow to the stator disc 5.

Fiber pad material adjacent the respective conductor body 9 and having high concentration of fiber pad material (having high content of fibers) does not conduct the primary electric current as well as a fiber pad material having low concentration of fiber pad material (having low content of fibers).

The electrical resistivity values and/or conductivity values and/or impedance values of measured primary electric current, being fed through the fiber pad material from the respective first electric surface 10 of the respective conductor body 9 one by one to the second electric contact surface 12, are values that are correlated with pre-determined fiber pad material concentration values, which have been empirically determined and being executed by the processor 23.

The processor 23 is configured to determinate and/or present fiber pad material concentration values for each position of the respective conductor body 9, and also a fiber concentration profile.

The processor 23 regulates the feeding rate of fibers and the feeding of adjusted amount of added water for mixing the fiber pad material, which will be grinded in the grinding gap 19.

The mixture constituting the fiber pad material may be achieved by controlling and regulating a control valve 31 of a water supply line 33.

FIG. 2 illustrates a stator disc 5 comprising a fiber concentration profile measuring sensor 1 according to a second example. The fibers and water being fed into a grinding gap 19 formed by a stationary grinding surface 13 of the stator disc 5 and a rotary grinding surface (not shown). The mixture is fed through a centre hole 35 of the stator disc 5 and being grinded into fiber pad material 37.

The fiber concentration profile measuring sensor 1 may be mounted at the stator disc 5 in such way that the conductor bodies 9 are in line with the radial direction and along the radius r of the stator disc 5.

The fiber concentration profile measuring sensor 1 detects the fiber concentration of the fiber pad material for each position of the respective conductor body 9 by detection of the change in electrical resistivity/impedance of the primary electric current fed through the fiber pad.

FIG. 3 illustrates a fiber concentration profile measuring sensor 1 according to a third example of the invention.

The area A of respective first electric contact surface 10 of each conductor body 9, the width of the grinding gap (not shown) being designated with w in the formula below, and the actual electrical resistivity $\rho$ of the fiber pad material will determine the fiber concentration of the fiber pad material.

A processor (not shown) calculates the fiber concentration of the fiber pad material for each position of the respective conductor body from the detection of the actual electrical resistivity $\rho$ of the fiber pad material in each position.

The processor (not shown) may calculate the fiber concentration profile of the fiber pad material in the grinding gap from the fiber concentration of the fiber pad material for each position along the radius of the refiner disc.

Each conductor body 9 may be fed with the primary electric current one by one, which is achieved by individually controlled electronic switching devices of a secondary circuit, which will be described further below.

The electrical resistance R of the fiber pad material is measured continuously at each position of the respective conductor body 9, through which the primary electric current is fed, one by one.

The formula for determining the resistance R is

$$R = \rho \cdot w / A$$

where R is resistance, $\rho$ is resistivity, w is the width of the grinding gap and A is the area of the respective first electric contact surface of the respective conductor body.

The detection of variations of the resistance R correlates to altered fiber concentration of the fiber pad material, which fiber concentration is calculated by the processor also executing a fiber concentration profile from detected fiber concentration and actual resistivity in each position.

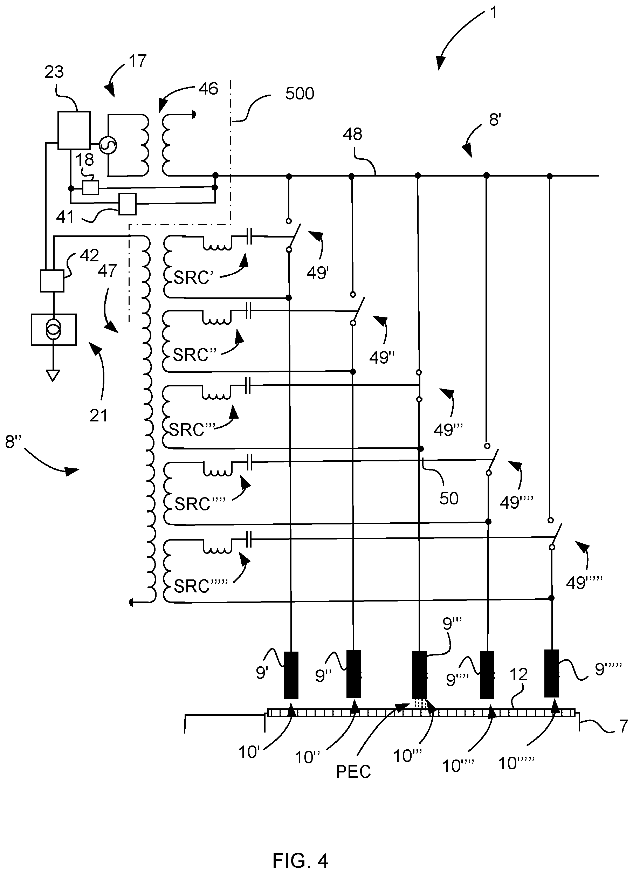

FIG. 4 illustrates a fiber concentration profile measuring sensor 1 according to a fourth example of the invention. A primary electric circuit 8' comprises a first conductor 9', a second conductor 9", a third conductor 9‴, a fourth conductor 9⁗ and a fifth conductor 9⁗′.

A primary AC power source 17 is configured to feed a primary electric current PEC to the primary electric circuit 8' and the respective conductor. The primary AC power source 17 is provided with a primary frequency generator 41 for generating a frequency of e.g. 50 kHz of the primary electric current.

The fiber concentration profile measuring sensor 1 further may comprises a processor 23 configured to execute detected resistance/impedance values of the primary electric current PEC into a calculation of a fiber concentration profile of the fiber pad material being grinded.

The primary AC power source 17 comprises an impedance measuring instrument 18 and a primary transformer 46. The primary AC power source 17 is coupled to the respective conductor via a primary conductor wire arrangement 48.

The first conductor 9' is coupled to the primary AC power source 17 via a first switch 49' (e.g. a semiconductor relay or solid-state relay). The second conductor 9" is coupled to the primary AC power source 17 via a second switch 49" (e.g. a semiconductor relay or solid-state relay). The third conductor 9‴ is coupled to the primary AC power source 17 via a third switch 49‴ (e.g. a semiconductor relay or solid-state relay). The fourth conductor 9⁗ is coupled to the primary AC power source 17 via a fourth switch 49⁗ (e.g. a semiconductor relay or solid-state relay). The fifth conductor 9⁗′ is coupled to the primary AC power source 17 via a fifth switch 49⁗′ (e.g. a semiconductor relay or solid-state relay).

The respective conductor exhibits a first electric contact surface 10', 10", 10‴, 10⁗, 10⁗′ configured to transfer the primary electric current PEC from the primary AC power source 17 to a second electric contact surface 12 of a rotary grinding disc 7 via a fiber pad material being pulped.

Each conductor 9', 9", 9‴, 9⁗, 9⁗′ being coupled in series with the respective switch 49', 49", 49‴, 49⁗, 49⁗′, each of which is configured to switch between on- and off-state for feeding or not feeding the primary electric current PEC to the associated conductor.

A secondary electric circuit 8" comprises a secondary power source 21, configured to generate a secondary electric current injected with a range of frequencies, e.g. 100-400 kHz, by means of a secondary signal generator 42. The secondary signal generator 42 is thus configured to generate a frequency sweep from e.g. 100 - 400 kHz to the secondary electric current.

The secondary signal generator 42 alternatively generates electrical output waveforms over a pre-determined range of frequencies and the secondary power source 21 is controlled by a frequency sweep controller (not shown) as to execute the frequency sweep. The secondary power source 21 comprises a secondary transformer 47.

The secondary power source 21 is coupled to the respective switch via a secondary conductor wire arrangement 50.

The secondary power source 21 is coupled to the first switch 49' via a first series resonance circuit SRC' being individually adapted to give free passage for the secondary electric current at 150 kHz providing an on-state of the first switch 49' for feeding the primary electric current PEC to the first conductor 9'.

The secondary power source 21 is coupled to the second switch 49" via a second series resonance circuit SRC" being individually adapted to give free passage for the secondary electric current at 200 kHz providing an on-state of the second switch 49" for feeding the primary electric current PEC to the second conductor 9".

The secondary power source 21 is coupled to the third switch 49‴ via a third series resonance circuit SRC‴ being individually adapted to give free passage for the secondary electric current at 250 kHz providing an on-state of the third switch 49‴ for feeding the primary electric current PEC to the third conductor 9‴. In the FIG. 4 is shown that the secondary electric current momentary is injected with a frequency of 250 kHz, whereby secondary electric current passes the third series resonance circuit SRC‴ and closes the third switch 49‴ so that momentary the primary electric current PEC passes the fiber pad material at the position of the third conductor 91‴.

The secondary power source 21 is coupled to the fourth switch 49⁗ via a fourth series resonance circuit SRC⁗ being individually adapted to give free passage for the secondary electric current at 300 kHz providing an on-state of the fourth switch 49⁗ for feeding the primary electric current PEC to the fourth conductor 9⁗.

The secondary power source 21 is coupled to the fifth switch 49″″ via a fifth series resonance circuit SRC″″ being individually adapted to give free passage for the secondary electric current at 350 kHz providing an on-state of the fifth switch 49″″ for feeding the primary electric current PEC to the fifth conductor 9″″.

By sweeping the frequency (e.g. 150-350 kHz) of the secondary electric current in the secondary circuit 8″, the specific electrical resistivity and/or conductivity and/or impedance of the primary electric current PEC fed through the fiber pad material in different positions along the radius of the refiner disc can be measured by the impedance measuring instrument 18 of the primary circuit 8′ providing impedance values to the control unit for converting the impedance values into actual fiber concentration profile values.

The respective first electrical contact surface 10, 10″, 10‴, 10″″, 10″″″ of each conductor being arranged at a specific distance from the second electric contact surface 12 of the opposite rotor disc 7.

The first series resonance circuit SRC′ permits passage of the secondary electric current at a first frequency and the other series resonance circuits close passage of the secondary electric current at said first frequency.

The second series resonance circuit SRC″ permits passage of the secondary electric current at a second frequency and the other series resonance circuits close passage of the secondary electric current at said second frequency, and so on, until the fifth series resonance circuit SRC″″ closes the fifth switch 49″″, thereafter the procedure is repeated.

The respective conductor body may be connected in series with the respective electronic switching device, each of which is configured to close or open (on-state or off-state) dependent on the control signal (to give free passage of current or hinder passage of current) from the respective series resonance circuits. These series connections may be coupled in parallel to the primary power source 17.

Closing a specific switch may be made for permitting the primary electric current to pass (by means of a specific series resonance circuit), whereas the other switches are remained open (i.e. not closed by the other series resonance circuits) for disconnecting the other conductor bodies from the primary AC power source.

In such way is achieved that there is no need to us an electric current at high and low bandwidth. Such electric current at high and low bandwidth may otherwise affect the performance of the fiber concentration profile measuring apparatus in view of less correct impedance/resistance values.

The primary AC power source may be configured to generate one single fixed frequency, which will produce a stable primary electric current, which in turn will produce reliable impedance values of the primary electric current passing the conductor bodies one by one.

By using a primary AC power source providing a primary electric current comprising a fixed frequency (e.g. 50 kHz), there will be a stable current to be measured in regard to the resistance or impedance.

The processor 23 of the fiber concentration profile measuring sensor 1 is pre-programmed with identification and/or position parameters associated with the identity of each relative position of the respective conductor body at the refiner disc.

By the sweeping of the frequency of the secondary electric current and by the pre-programmed identification/position parameters, the processor 23 is able to detect the resistance/impedance values for each "earmarked" conductor body along the radius of the stator disc.

Cost-effective mounting and service maintenance is achieved by that the number of lead wires running from the fiber concentration profile measuring apparatus is reduced at the same time as a stable primary electric current injected with a pre-set and fixed frequency (e.g. 50 kHz) being fed to each conductor body one by one.

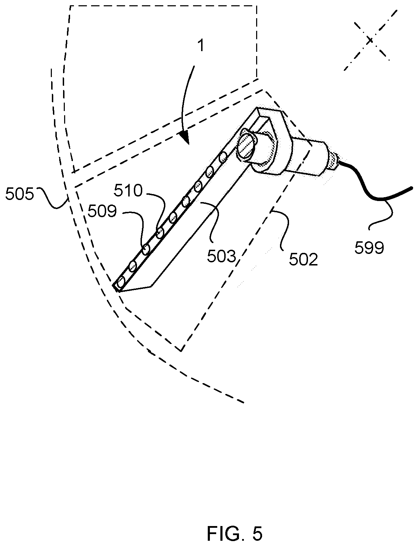

FIG. 5 illustrates a fiber concentration profile measuring sensor 1 according to a fifth example. The fiber concentration profile measuring sensor 1 is mounted on a disc segment 502 of a stator disc 505 and comprises a plurality of conductors 509 being arranged in line within a housing 503. A first electric contact surface 510 of each conductor 9 is exposed and faces an opposite refiner disc (not shown) also serving as a second electric contact surface. A cable 599 comprising only few lead wires runs from the fiber concentration profile measuring apparatus 1. An interface 500 shown in FIG. 4 may be positioned as illustrated for fulfilling the reduction of lead wires coming from the housing 503.

The fiber concentration profile measuring sensor 1 may comprise an adjustable gap sensor AGS configured to detect changes of the width of the grinding gap for compensating and calculating the fiber concentration of the fiber pad material. In such way is provided a multifunctional sensor, which is adapted for cost-effective production of pulp material.

The present invention is of course not in any way restricted to the preferred embodiments described above, but many possibilities to modifications, or combinations of the described embodiments, thereof should be apparent to a person with ordinary skill in the art without departing from the basic idea of the invention as defined in the appended claims. The fiber pad material preferably comprises cellulose fibers for making paper pulp. The control unit may be configured to present a graph schematically illustrating magnitude plots generated by the respective conductor body, the positions of which are determined over the radius of the refiner disc. The control unit may be configured to execute a profile graph of the temperature of the fiber pad material along the radial direction of the refiner disc.

The temperature may be derived by the control unit from signals fed from a resistance temperature detector (e.g. a PT100 element).

Variations in resistivity over time for different positions of the radius of the refiner disc are parameters that are used for deriving the temperature profile. The computation of the profile graph is made by the control unit adapted to detect the alteration of the electrical resistivity and/or conductivity of the fiber pad material. The fiber pad material may serve as a conductor by itself and may exhibit a certain conductivity/electrical resistivity depending upon the actual fiber concentration and/or the actual moisture content and/or upon the actual gaseous or liquid state of the water or fluid content or other gaseous phase of the fiber pad material. Each position of the respective conductor body may be fixed relative one of the refiner discs and each position may be registered in a coordinate system or other position system by means of the control unit. The secondary power source may comprise a semiconductor or a transistor. The electronic switching device may comprise a solid-state electronic switch device or solid-state relay that comprises a transistor switch or sensor, which respond to a specific frequency of the secondary electric current (control signal). Each conductor body may be fed with the primary electric current two by two.

**Claims**

1. A fiber concentration profile measuring apparatus (1) comprising a plurality of conductor bodies (9, 509), configured to be coupled to a primary AC power source (17) comprising an impedance measuring unit (18), the respective conductor body (9, 509) exhibits a first electric contact surface (10) configured to transfer a primary electric current (PEC) from the primary AC power source (17) to a second electric contact surface (12) via a fiber pad material being pulped, **characterized in that**

   - each conductor body (9, 509) is coupled in series with a respective electronic switching device (49', 49", 49‴, 49"", 49‴""), each of which is configured to switch between on- and off-state for feeding or not feeding the primary electric current (PEC) to the associated conductor body (9, 509); and
   - a secondary power source (21), configured to generate a secondary electric current injected with a range of frequencies, is coupled to each electronic switching device (49', 49", 49‴, 49"", 49‴"") via a respective series resonance circuit (SRC', SRC", SRC‴, SRC"", SRC‴""), each being individually adapted to give free passage for the secondary electric current at a certain frequency providing said on-state.

2. The fiber concentration profile measuring apparatus (1) according to claim 1, **wherein** a first series resonance circuit (SRC'), at a first frequency, is configured to give free passage for the secondary electric current to a first electronic switching device (49') and a second series resonance circuit (SRC'), at a second frequency, is configured to give free passage for the secondary current to a second electronic switching device (49").

3. The fiber concentration profile measuring apparatus (1) according to claim 1 or 2,
   **wherein** the secondary power source (21) is provided with a frequency generator (42) for generating a range of varying frequencies of the secondary electric current.

4. The fiber concentration profile measuring apparatus (1) according to any of claims 1 to 3, **wherein** the second electric contact surface (10) is formed by a rotor disc (7) of a refiner apparatus (2).

5. The fiber concentration profile measuring apparatus (1) according to any of the preceding claims, **wherein** the apparatus (1) comprises;

   - a first conductor body (9') coupled to the primary AC power source (17) via a first electronic switching device (49');
   - a second conductor body (9") coupled to the primary AC power source (17) via a second electronic switching device (49"); and
   - a third conductor body (9‴) coupled to the primary AC power source (17) via a third electronic switching device (49‴).

6. The fiber concentration profile measuring apparatus (1) according to any of the preceding claims, **wherein**

   - the first conductor body (9') exhibits a first electric contact surface (10') adapted to transfer the primary electric current (PEC) from the primary AC power source (17) to the second electric contact surface (12) via the fiber pad material being pulped;
   - the second conductor body (9") exhibits a second electric contact surface (10") adapted to transfer the primary electric current (PEC) from the primary AC power source (17) to the second electric contact surface (12) via the fiber pad material being pulped; and
   - the third conductor body (9‴) exhibits a third electric contact surface (10‴) adapted to transfer the primary electric current (PEC) from the primary AC power source (17) to the second electric contact surface (12) via the fiber pad material being pulped.

7. The fiber concentration profile measuring apparatus (1) according to any of the preceding claims, **wherein**

- the first conductor body (9') is coupled in series with the first electronic switching device (49');
- the second conductor body (9") is coupled in series with the second electronic switching device (49"); and
- the third conductor body (9‴) is coupled in series with the first third electronic switching device (49‴).

8. The fiber concentration profile measuring apparatus (1) according to any of the preceding claims, **wherein**

- the first electronic switching device (49') is configured to switch between first on-state and first off-state so that the primary AC power source (17) can feed or not feed the primary electric current (PEC) to the first conductor body (9');
- the second electronic switching device (49") is configured to switch between second on-state and second off-state so that the primary AC power source (17) can feed or not feed the primary electric current (PEC) to the second conductor body (9"); and
- the third electronic switching device (49‴) is configured to switch between third on-state and third off-state so that the primary AC power source (17) can feed or not feed the primary electric current (PEC) to the third conductor body (9‴).

9. The fiber concentration profile measuring apparatus (1) according to any of the preceding claims, **wherein** the apparatus (1) comprises a control unit (23) adapted to execute and determinate a fiber concentration profile of the fiber pad material being pulped from values of the electrical impedance of the primary electric current (PEC), which values being achieved by sweeping frequencies of the secondary electric current.

10. A refiner apparatus (2) comprising the fiber concentration profile measuring apparatus (1) according to any of the preceding claims.

**Patentansprüche**

1. Vorrichtung (1) zur Faserkonzentrationsprofilmessung, umfassend eine Vielzahl von Leiterkörpern (9, 509), die dazu konfiguriert ist, an eine Primär-AC-Leistungsquelle (17) gekoppelt zu werden, die eine Impedanzmesseinheit (18) umfasst, wobei der jeweilige Leiterkörper (9, 509) eine erste elektrische Kontaktfläche (10) aufweist, die dazu konfiguriert ist, einen elektrischen Primärstrom (PEC) von der Primär-AC-Leistungsquelle (17) über ein Faserkontaktstellenmaterial, das aufgeschlossen wird, an eine zweite elektrische Kontaktfläche (12) zu übertragen, **dadurch gekennzeichnet, dass**

- jeder Leiterkörper (9, 509) in Reihe mit einer jeweiligen elektronischen Schalteinrichtung (49', 49", 49‴, 49⁗, 49⁗') gekoppelt ist, wovon jede dazu konfiguriert ist, zwischen einem eingeschalteten und ausgeschalteten Zustand zu schalten, um den elektrischen Primärstrom (PEC) in den zugeordneten Leiterkörper (9, 509) einzuspeisen oder nicht einzuspeisen; und
- eine Sekundärleistungsquelle (21), die dazu konfiguriert ist, einen elektrischen Sekundärstrom zu generieren, der mit einem Bereich von Frequenzen injiziert wird, über eine jeweilige Reihenresonanzschaltung (SRC', SRC", SRC‴, SRC⁗, SRC⁗') an jede elektronische Schalteinrichtung (49', 49", 49‴, 49⁗, 49⁗') gekoppelt ist, wobei jede individuell dazu ausgelegt ist, bei einer gewissen Frequenz, die den eingeschalteten Zustand bereitstellt, freien Durchgang für den elektrischen Sekundärstrom zu gewähren.

2. Vorrichtung (1) zur Faserkonzentrationsprofilmessung nach Anspruch 1, wobei eine erste Reihenresonanzschaltung (SRC') bei einer ersten Frequenz dazu konfiguriert ist, freien Durchgang für den elektrischen Sekundärstrom zu einer ersten elektronischen Schalteinrichtung (49') zu gewähren, und eine zweite Reihenresonanzschaltung (SRC') bei einer zweiten Frequenz dazu konfiguriert ist, freien Durchgang für den Sekundärstrom zu einer zweiten elektronischen Schalteinrichtung (49") zu gewähren.

3. Vorrichtung (1) zur Faserkonzentrationsprofilmessung nach Anspruch 1 oder 2, wobei die Sekundärleistungsquelle (21) mit einem Frequenzgenerator (42) zum Generieren eines Bereichs von variierenden Frequenzen des elektrischen Sekundärstroms bereitgestellt ist.

4. Vorrichtung (1) zur Faserkonzentrationsprofilmessung nach einem der Ansprüche 1 bis 3, wobei die zweite elektrische Kontaktfläche (10) durch eine Rotorscheibe (7) einer Refiner-Vorrichtung (2) gebildet ist.

5. Vorrichtung (1) zur Faserkonzentrationsprofilmessung nach einem der vorhergehenden Ansprüche, wobei die Vorrichtung (1) Folgendes umfasst:

- einen ersten Leiterkörper (9'), der über eine erste elektronische Schalteinrichtung (49') an die Primär-AC-Leistungsquelle (17) gekoppelt ist;

- einen zweiten Leiterkörper (9"), der über eine zweite elektronische Schalteinrichtung (49") an die Primär-AC-Leistungsquelle (17) gekoppelt ist; und

- einen dritten Leiterkörper (9‴), der über eine dritte elektronische Schalteinrichtung (49‴) an die Primär-AC-Leistungsquelle (17) gekoppelt ist.

6. Vorrichtung (1) zur Faserkonzentrationsprofilmessung nach einem der vorhergehenden Ansprüche, wobei

- der erste Leiterkörper (9') eine erste elektrische Kontaktfläche (10') aufweist, die dazu ausgelegt ist, den elektrischen Primärstrom (PEC) von der Primär-AC-Leistungsquelle (17) über das Faserkontaktstellenmaterial, das aufgeschlossen wird, an die zweite elektrische Kontaktfläche (12) zu übertragen;

- der zweite Leiterkörper (9") eine zweite elektrische Kontaktfläche (10") aufweist, die dazu ausgelegt ist, den elektrischen Primärstrom (PEC) von der Primär-AC-Leistungsquelle (17) über das Faserkontaktstellenmaterial, das aufgeschlossen wird, an die zweite elektrische Kontaktfläche (12) zu übertragen; und

- der dritte Leiterkörper (9‴) eine dritte elektrische Kontaktfläche (10‴) aufweist, die dazu ausgelegt ist, den elektrischen Primärstrom (PEC) von der Primär-AC-Leistungsquelle (17) über das Faserkontaktstellenmaterial, das aufgeschlossen wird, an die zweite elektrische Kontaktfläche (12) zu übertragen.

7. Vorrichtung (1) zur Faserkonzentrationsprofilmessung nach einem der vorhergehenden Ansprüche, wobei

- der erste Leiterkörper (9') in Reihe mit der ersten elektronischen Schalteinrichtung (49') gekoppelt ist;

- der zweite Leiterkörper (9") in Reihe mit der zweiten elektronischen Schalteinrichtung (49") gekoppelt ist; und

- der dritte Leiterkörper (9‴) in Reihe mit der ersten dritten elektronischen Schalteinrichtung (49‴) gekoppelt ist.

8. Vorrichtung (1) zur Faserkonzentrationsprofilmessung nach einem der vorhergehenden Ansprüche, wobei

- die erste elektronische Schalteinrichtung (49') dazu konfiguriert ist, zwischen einem ersten eingeschalteten Zustand und einem ersten ausgeschalteten Zustand zu schalten, sodass die Primär-AC-Leistungsquelle (17) den elektrischen Primärstrom (PEC) in den ersten Leiterkörper (9') einspeisen kann oder nicht einspeisen kann;

- die zweite elektronische Schalteinrichtung (49") dazu konfiguriert ist, zwischen einem zweiten eingeschalteten Zustand und einem zweiten ausgeschalteten Zustand zu schalten, sodass die Primär-AC-Leistungsquelle (17) den elektrischen Primärstrom (PEC) in den zweiten Leiterkörper (9") einspeisen kann oder nicht einspeisen kann; und

- die dritte elektronische Schalteinrichtung (49‴) dazu konfiguriert ist, zwischen einem dritten eingeschalteten Zustand und einem dritten ausgeschalteten Zustand zu schalten, sodass die Primär-AC-Leistungsquelle (17) den elektrischen Primärstrom (PEC) in den dritten Leiterkörper (9‴) einspeisen kann oder nicht einspeisen kann.

9. Vorrichtung (1) zur Faserkonzentrationsprofilmessung nach einem der vorhergehenden Ansprüche, wobei die Vorrichtung (1) eine Steuereinheit (23) umfasst, die dazu ausgelegt ist, ausgeführt zu werden und ein Faserkonzentrationsprofil des Faserkontaktstellenmaterials, das aufgeschlossen wird, aus Werten der elektrischen Impedanz des elektrischen Primärstroms (PEC) zu bestimmen, wobei die Werte durch Wobbeln von Frequenzen des elektrischen Sekundärstroms erzielt werden.

10. Refiner-Vorrichtung (2), umfassend die Vorrichtung (1) zur Faserkonzentrationsprofilmessung nach einem der vorhergehenden Ansprüche.

## Revendications

1. Appareil de mesure de profil de concentration de fibres (1) comprenant une pluralité de corps conducteurs (9, 509), configuré pour être couplé à une source d'alimentation CA primaire (17) comprenant une unité de mesure d'impédance (18), le corps conducteur (9, 509) respectif présente une première surface de contact électrique (10) configurée pour transférer un courant électrique primaire (PEC) de la source d'alimentation CA primaire (17) à une deuxième surface de contact électrique (12) via un matériau de tampon de fibres à pâte de papier, **caractérisé en ce que**

- chaque corps conducteur (9, 509) est couplé en série à un dispositif de commutation électronique (49', 49'', 49''', 49'''', 49''''') respectif, dont chacun est configuré pour commuter entre l'état passant et l'état bloqué pour fournir ou non le courant électrique primaire (PEC) au corps conducteur (9, 509) associé ; et

- une source d'alimentation secondaire (21), configurée pour générer un courant électrique secondaire injecté avec une plage de fréquences, est couplée à chaque dispositif de commutation électronique (49', 49'', 49''', 49'''', 49 ''''') via un circuit de résonance série (SRC', SRC'', SRC''', SRC'''', SRC''''') respectif, chacun étant adapté individuellement pour laisser libre passage au courant électrique secondaire à une certaine fréquence fournissant ledit état passant.

2. Appareil de mesure de profil de concentration de fibres (1) selon la revendication 1, dans lequel un premier circuit de résonance série (SRC'), à une première fréquence, est configuré pour laisser libre passage au courant électrique secondaire vers un premier dispositif de commutation électronique (49') et un second circuit de résonance série (SRC'), à une seconde fréquence, est configuré pour laisser libre passage au courant secondaire vers un deuxième dispositif de commutation électronique (49'').

3. Appareil de mesure de profil de concentration de fibres (1) selon la revendication 1 ou 2, dans lequel la source d'alimentation secondaire (21) est pourvue d'un générateur de fréquence (42) pour générer une plage de fréquences variables du courant électrique secondaire.

4. Appareil de mesure de profil de concentration de fibres (1) selon l'une quelconque des revendications 1 à 3, dans lequel la deuxième surface de contact électrique (10) est formée par un disque de rotor (7) d'un appareil raffineur (2) .

5. Appareil de mesure de profil de concentration de fibres (1) selon l'une quelconque des revendications précédentes, dans lequel l'appareil (1) comprend :

- un premier corps conducteur (9') couplé à la source d'alimentation CA primaire (17) via un premier dispositif de commutation électronique (49') ;
- un deuxième corps conducteur (9'') couplé à la source d'alimentation CA primaire (17) via un deuxième dispositif de commutation électronique (49'') ; et
- un troisième corps conducteur (9''') couplé à la source d'alimentation CA primaire (17) via un troisième dispositif de commutation électronique (49''').

6. Appareil de mesure de profil de concentration de fibres (1) selon l'une quelconque des revendications précédentes, dans lequel

- le premier corps conducteur (9') présente une première surface de contact électrique (10')

adaptée pour transférer le courant électrique primaire (PEC) de la source d'alimentation CA primaire (17) à la deuxième surface de contact électrique (12) via le matériau de tampon de fibres à pâte à papier ;
- le deuxième corps conducteur (9'') présente une deuxième surface de contact électrique (10'') adaptée pour transférer le courant électrique primaire (PEC) de la source d'alimentation CA primaire (17) à la deuxième surface de contact électrique (12) via le matériau de tampon de fibres à pâte à papier ; et
- le troisième corps conducteur (9''') présente une troisième surface de contact électrique (10''') adaptée pour transférer le courant électrique primaire (PEC) de la source d'alimentation CA primaire (17) à la deuxième surface de contact électrique (12) via le matériau de tampon de fibres à pâte à papier.

7. Appareil de mesure de profil de concentration de fibres (1) selon l'une quelconque des revendications précédentes, dans lequel

- le premier corps conducteur (9') est couplé en série au premier dispositif de commutation électronique (49') ;
- le deuxième corps conducteur (9'') est couplé en série au deuxième dispositif électronique de commutation (49'') ; et
- le troisième corps conducteur (9''') est couplé en série au premier troisième dispositif de commutation électronique (49''').

8. Appareil de mesure de profil de concentration de fibres (1) selon l'une quelconque des revendications précédentes, dans lequel

- le premier dispositif de commutation électronique (49') est configuré pour commuter entre le premier état passant et le premier état bloqué de sorte que la source d'alimentation CA primaire (17) peut amener ou non le courant électrique primaire (PEC) vers le premier conducteur corps (9') ;
- le deuxième dispositif de commutation électronique (49'') est configuré pour commuter entre le deuxième état passant et le deuxième état bloqué de sorte que la source d'alimentation CA primaire (17) peut amener ou non le courant électrique primaire (PEC) au deuxième corps conducteur (9'') ; et
- le troisième dispositif de commutation électronique (49''') est configuré pour commuter entre le troisième état passant et le troisième état bloqué de sorte que la source d'alimentation CA primaire (17) peut amener ou non le courant électrique primaire (PEC) au troisième corps conducteur (9''').

9.  Appareil de mesure de profil de concentration de fibres (1) selon l'une quelconque des revendications précédentes, dans lequel l'appareil (1) comprend une unité de commande (23) adaptée pour exécuter et déterminer un profil de concentration de fibres du matériau de tampon de fibres à pâte à papier à partir de valeurs de l'impédance électrique du courant électrique primaire (PEC), lesquelles valeurs sont obtenues par balayage des fréquences du courant électrique secondaire.

10. Appareil raffineur (2) comprenant l'appareil de mesure de profil de concentration de fibres (1) selon l'une quelconque des revendications précédentes.

FIG. 1

FIG. 3

FIG. 2

FIG. 4

FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015065268 A1
- US 6087837 A
- EP 1132518 A